# FASCICULE DE BREVET EUROPEEN

(11) **EP 1 095 670 B1**
(45) Date de publication et mention de la délivrance du brevet: **07.05.2008**
(21) Numéro de dépôt: 99121611.0
(22) Date de dépôt: 29.10.1999
(51) Int. Cl.: A61N 1/36

(54) **Stimulateur électrique neuromusculaire avec mesure des réponses musculaires aux impulsions électriques de stimulation**
Neuromuskuläres Stimulationsgerät mit Aufnahme der Muskelreaktion auf den elektrischen Stimulationsimpuls
Neuromuscular stimulator with measurement of the muscular response on the electrical stimulation impulse

(43) Date de publication de la demande: 02.05.2001
(73) Titulaire: COMPEX MEDICAL S.A, 1024 Ecublens (CH)
(72) Inventeur: Rigaux, Pierre, 4020 Liège (BE); Buhlmann, Félix, 1018 Lausanne (CH); Müller, Pierre-Yves, 1248 Hermance (CH)
(74) Mandataire: Besse, François

(56) Documents cités:
- EP-A- 0 436 121
- WO-A-95/10323
- WO-A-99/19019
- US-A- 4 524 774
- US-A- 4 580 339
- US-A- 4 805 636
- US-A- 5 070 873
- US-A- 5 131 401
- US-A- 5 355 883
- US-A- 5 507 788
- US-A- 5 540 735
- US-A- 5 851 191

## Description

L'invention concerne un stimulateur électrique neuromusculaire avec mesure des réactions musculaires générées par des impulsions électriques. Le stimulateur comprend un générateur d'impulsions électriques disposé dans un boîtier, au moins une paire d'électrodes de stimulation destinées à être posées sur la peau d'un utilisateur au niveau des points moteurs des muscles à stimuler, chaque électrode étant reliée à une extrémité d'un câble électrique dont l'autre extrémité est raccordée au boîtier pour recevoir les impulsions électriques du générateur, au moins un capteur sensible à des réactions musculaires provoquées par les impulsions électriques de stimulation et agencé pour transmettre des signaux électriques de mesure représentatifs desdites réactions musculaires à des moyens électroniques dans le boîter du stimulateur.

L'invention concerne également un câble électrique pour un stimulateur électrique neuromusculaire.

Le capteur fournit des informations sur les réactions musculaires utiles notamment pour connaître l'état de fatigue des muscles stimulés électriquement. Les mesures obtenues du capteur permettent d'adapter les paramètres des impulsions électriques de stimulation que ce soit manuellement à l'aide de la vision sur un écran de la forme des signaux reçus par le capteur ou automatiquement par adaptation des paramètres électriques de stimulation en fonction de la fatigue musculaire. L'adaptation des paramètres consiste à corriger soit la fréquence des impulsions, soit l'amplitude ou la durée des impulsions de tension ou de courant, soit les durées de contraction et de relaxation du muscle, soit le nombre de cycles de contraction/relaxation, soit une association quelconque des paramètres précédents.

La stimulation électrique ou électrostimulation a pour but de commander un travail aux muscles par le biais d'impulsions électriques de tension ou de courant en fonction de paramètres programmés. Chaque impulsion de courant ou de tension fournit une excitation au niveau des fibres nerveuses qui commandent les fibres musculaires via la plaque motrice. Cette excitation donne lieu à une réponse mécanique élémentaire du muscle appelée secousse d'une durée de l'ordre de 0.1s.

L'impulsion de tension ou de courant est répétée dans le temps selon une fréquence ajustable. Si cette fréquence est basse, par exemple de 10 Hz, la puissance de travail des muscles est légère, tandis que pour une fréquence élevée, par exemple de 100 Hz, la puissance de travail des fibres musculaires stimulées est très élevée. Cette puissance très élevée correspond à une puissante contraction tétanique. Les secousses des fibres musculaires ne peuvent plus à cette fréquence élevée être séparées après chaque impulsion ce qui fait qu'il se produit une sommation temporelle des secousses qui conduit à une contraction tétanique.

Si les muscles stimulés sont stimulés à fréquence élevée, ils auront tendance à se fatiguer. Dans ce cas, la séance d'entraînement consiste à imposer une alternance de périodes de contraction et de périodes de repos. La phase de repos permet aux fibres de se relâcher et de récupérer avant la prochaine phase de contraction.

Dans le domaine médical, les stimulateurs électriques sont utilisés pour l'aide à des personnes handicapées ou accidentées de façon à pallier aux déficiences d'activité musculaire ou à leur permettre une réhabilitation de leur musculature meurtrie. Des impulsions électriques de courant ou de tension sont transmises auxdits muscles via les électrodes posées à même la peau ou sous-cutanées afin de les faire travailler passivement. Des mesures de la réaction musculaire causée par l'excitation évoquée électriquement permettent d'adapter parfois les impulsions électriques à transmettre aux électrodes en fonction du niveau de l'amplitude électrique ou mécanique mesurée sur les muscles innervés sans pour autant fatiguer exagérément les muscles stimulés. Cette adaptation de paramètres électriques du stimulateur sert notamment aux personnes handicapées, voire accidentées, pour éviter qu'elles soient dans l'obligation de demander continuellement une aide externe lorsqu'elles doivent bouger l'un ou l'autre de leurs membres déficients.

Un tel stimulateur est montré dans le document US 5070873 dans lequel une boucle de réglage des impulsions électriques à fournir aux muscles pour leur donner une motricité suffisante est décrite. Dans une première phase, des capteurs électromyographiques détectent l'activité musculaire volontaire qui dans le cas d'une personne handicapée est déficiente. La mesure de tension obtenue par les capteurs représentent l'état faible de contraction des muscles activés ce qui conduit à adapter les impulsions électriques du générateur d'impulsions pour envoyer vers les nerfs-moteurs des muscles des impulsions de tension adaptées à la réaction attendue notamment pour permettre un automatisme dans le déroulement des mouvements souhaités par l'handicapé.

Les capteurs électromyographiques peuvent être séparés des électrodes de stimulation, mais également combinés avec elles. Dans ce dernier cas de figure, une troisième électrode est nécessaire. Si on utilise la même surface active de l'électrode comme électrode de stimulation et comme capteur, cela implique de gérer de manière non évidente les signaux provenant du capteur suite aux impulsions électriques envoyées sur l'électrode.

La combinaison du capteur avec l'électrode nécessite d'envoyer des impulsions rectangulaires de tension biphasiques pour effectuer des mesures par le capteur. Il est à noter que dans ce cas de figure, pour des impulsions de tension, le courant de stimulation fourni dépend de l'impédance de l'électrode et de la peau. Cette impédance n'est pas la même d'une personne à l'autre, ou peut varier rapidement au cours du temps chez une même personne, ce qui conduit à des réactions du muscle différentes pour des impulsions de tension identiques envoyées aux électrodes.

L'emploi d'un générateur d'impulsions de courant permet de s'affranchir des inconvénients d'un générateur d'impulsions de tension, car l'impulsion est maintenue constante quelle que soit l'impédance de la peau et de l'électrode, et ainsi permettre de maintenir le même nombre de fibres recrutées pour la stimulation.

Un inconvénient de cette combinaison des surfaces actives du capteur et de l'électrode réside dans le fait qu'après la séquence d'impulsions de tension biphasiques envoyées, il subsiste une tension résiduelle qui peut valoir une dizaine de volts, alors que la tension de mesure tirée des muscles par le capteur est de l'ordre de quelques millivolts. Il est donc nécessaire d'atténuer cette tension résiduelle pour pouvoir faire une mesure précise notamment de l'état de fatigue des muscles stimulés. C'est pourquoi, des capteurs séparés des électrodes fournissent de meilleurs résultats que ceux combinés comme décrit ci-dessus.

Dans le document FR 2 425 865, il est décrit également un stimulateur électrique de muscles à commande bioélectrique. Un générateur de fréquence porteuse fournit un signal électrique aux muscles à stimuler qui est adapté en fonction de l'activité bioélectrique des muscles innervés. Avec cette régulation des impulsions électriques en fonction de la réaction mesurée des muscles, ce stimulateur offre une large palette d'utilisations. Il permet notamment de créer un certain automatisme moteur de mouvements par exemple lors d'un entraînement sportif ou pour l'aide à des personnes handicapées.

Les capteurs de mesure sont du type électromyographique et peuvent être aussi combinés avec les électrodes de stimulation, mais dans ce cas, comme les impulsions de tensions envoyées aux muscles sont principalement des tensions d'ordre sinusoïdal, cela ne pose pas trop de problème de tirer les signaux EMG provenant des mêmes électrodes de stimulation à l'aide de filtres, ce qui n'est pas le cas avec des impulsions rectangulaires de tension.

Les moyens de mesure de la contraction musculaire pour fournir une information sur l'état de réaction des muscles stimulés peuvent être exécutés de multiples façons. La mesure peut être soit électrique à l'aide de capteurs électromyographiques, soit mécanique suivie d'une conversion électrique à l'aide par exemple de capteurs acoustiques (microphones). Un tel agencement est montré dans le document US 4805636 où il s'agit de mesurer les vibrations des muscles qui se contractent.

Dans ce document, deux microphones sont placés à des endroits différents où les muscles innervés répondent mécaniquement par une secousse à l'impulsion de tension produite par un générateur d'impulsions électriques. Un circuit de retour prend en compte les signaux de tension donnés par les deux microphones afin d'adapter la secousse ou les impulsions électriques que le générateur produit à destination des muscles.

Des jauges de contrainte comme autre type de capteur mécanique à conversion électrique peuvent être utilisées comme le décrit le document US 5507788. Les jauges de contrainte servent à mesure un couple développer par les muscles stimuler. Elles sont disposées distantes des électrodes de stimulation. Les signaux ainsi obtenus des jauges sont traités par une circuiterie dans le stimulateur afin d'adapter les paramètres de stimulation du générateur d'impulsions en fonction notamment de la fatigue musculaire.

L'emploi de jauges de contrainte ne peut s'appliquer que dans le cas où il est possible de pouvoir mesurer un couple de force. Un tel capteur n'est, par contre, pas approprié s'il s'agit de faire des mesures pour des muscles dorsaux ou pectoraux par exemple, qui n'entraînent pas de mouvement d'un segment.

Dans le document US 5 131 401, il est décrit également un stimulateur électrique neuromusculaire.

Un but que se propose de résoudre l'invention consiste à utiliser une structure combinant une électrode de stimulation avec un capteur de mesure des réactions musculaires en palliant les inconvénients des stimulateurs ci-dessus décrits.

Un autre but de l'invention consiste à permettre à un utilisateur de ne penser qu'à placer les électrodes sur les muscles comme pour un stimulateur électrique standard tout en lui fournissant en plus par les capteurs combinés avec les électrodes respectives des mesures sur les réactions musculaires aux endroits stimulés.

Les buts de l'invention sont atteints grâce au stimulateur comme indiqué ci-dessus qui se caractérise en ce que le capteur est lié mécaniquement à une des électrodes en étant placé dans la structure de celle-ci ou à l'extrémité d'un des câbles côté électrode, en étant placé dans un connecteur reliant le câble à l'électrode.

Les buts de l'invention sont également atteints grâce au câble électrique pour un stimulateur qui se caractérise en ce qu'une extrémité du câble côté électrode a un connecteur pour être relié à la structure d'une électrode de stimulation par des moyens de fixation amovible servant également de contact électrique pour la ou les surfaces actives de l'électrode, et en ce que le connecteur comprend au moins une partie d'un capteur de mesure des réactions musculaires.

Un avantage du stimulateur avec la combinaison électrode et capteur de mesure consiste à faciliter la mise en place desdits éléments par exemple pour l'entraînement passif d'un sportif à l'aide d'un tel stimulateur ou pour toutes autres applications. Le sportif sait où placer les électrodes sur les points moteurs des muscles qu'il souhaite entraîner. Au début de l'utilisation d'un tel stimulateur, il a dû apprendre à situer les points moteurs pour les muscles à entraîner. Par habitude, il sait facilement les poser aux endroits désirés et ainsi commencer sa séance d'entraînement.

L'adjonction supplémentaire d'un capteur avec l'électrode qu'il positionne ne lui posera aucun problème supplémentaire. En plus de la stimulation, il pourra se rendre compte de la fatigue des muscles stimulés par exemple sur un écran du stimulateur.

De même, si le stimulateur comprend des moyens de réception de signaux du capteur capable d'agir sur le générateur d'impulsions, les impulsions électriques de tension ou de courant envoyées aux électrodes de stimulation sont automatiquement adaptées en fonction de la fatigue musculaire évitant par la même occasion toute manipulation subséquente de l'utilisateur. Les signaux de mesure émis du capteur aux moyens de réception du stimulateur passent soit par un fil conducteur isolé dans la gaîne du câble électrique autre que le fil conducteur de l'électrode, soit à l'aide de moyens d'émission de signaux sans fil de connexion.

Un avantage d'employer un capteur du type électromyographique ou à conversion mécanique-électrique du type accéléromètre ou acoustique réside dans leur utilisation à n'importe quel muscle du corps. Des muscles dorsaux sont un des exemples de muscles dans lesquels la mesure de la réaction n'est pas possible à l'aide d'un capteur à jauge de contrainte ou plus généralement à l'aide d'un capteur de couple ou de force.

Un autre avantage dans l'utilisation d'un capteur électromyographique avec une électrode consiste à avoir deux surfaces actives, l'une pour le capteur et l'autre pour l'électrode. De sorte, on mesure les réponses musculaires en minimisant les perturbations provenant de la surface active de l'électrode de stimulation.

Un autre avantage du stimulateur selon l'invention consiste à minimiser le nombre d'électrodes combinées avec des capteurs de mesure nécessaires pour d'une part stimuler les muscles et d'autre part mesurer les réactions musculaires. Une paire d'électrodes combinées avec au moins un capteur suffit pour stimuler les muscles aux endroits désirés et donner les informations sur les réactions des muscles stimulés. Les fils conducteurs reliant le capteur et l'électrode respective sont regroupés dans un seul câble électrique. Les coûts de fabrication sont donc réduits au minimum.

Les buts, avantages et caractéristiques du stimulateur apparaîtront mieux, de manière non limitative, dans la description qui va suivre grâce à différentes formes d'exécution illustrées par les dessins sur lesquels :
Les figures 1a et 1b représentent le stimulateur avant et après la connexion des câbles électriques sur les électrodes posées sur la peau d'un utilisateur,
la figure 2 représente une coupe partielle d'une première forme d'exécution d'un connecteur de câble électrique avec un capteur de mesure intégré fixé sur une électrode de stimulation,
les figures 3a et 3b représentent une coupe partielle verticale et une vue de dessous d'une deuxième forme d'exécution d'un agencement d'un capteur électromyographique et d'une électrode,
la figure 4 représente une coupe partielle d'une troisième forme d'exécution d'un connecteur de câble électrique avec un capteur de mesure intégré fixé sur une électrode de stimulation, et
la figure 5 représente des diagrammes de signaux électriques envoyés aux électrodes et de la réponse musculaire.

Le stimulateur décrit ci-après se rapporte de préférence à un stimulateur utilisé dans le domaine sportif et de la rééducation où la stimulation des muscles sert à les entraîner passivement. Des impulsions rectangulaires de courant sont fournies aux électrodes 7 posées sur la peau au niveau des points moteurs des muscles à stimuler. En réponse à cette stimulation, les muscles se contractent générant une secousse mécanique. Comme décrit précédemment, il s'avère que des impulsions de courant sont préférées à des impulsions de tension, car on n'est pas dépendant de l'impédance variable de l'électrode et de la peau de la personne utilisant le stimulateur.

Les impulsions de courant s'enchaînent dans le temps à une fréquence donnée. Selon la fréquence de répétition des impulsions, les muscles n'ont pas le temps de se décontracter avant la prochaine impulsion ce qui augmente la puissance de travail des muscles, mais en contre partie, ils se fatiguent. Il est donc intéressant de connaître la fatigue des muscles stimulés pour être informé de l'état des muscles entraînés et également pour pouvoir tirer profit de cette mesure afin d'adapter de manière automatique les paramètres de stimulation.

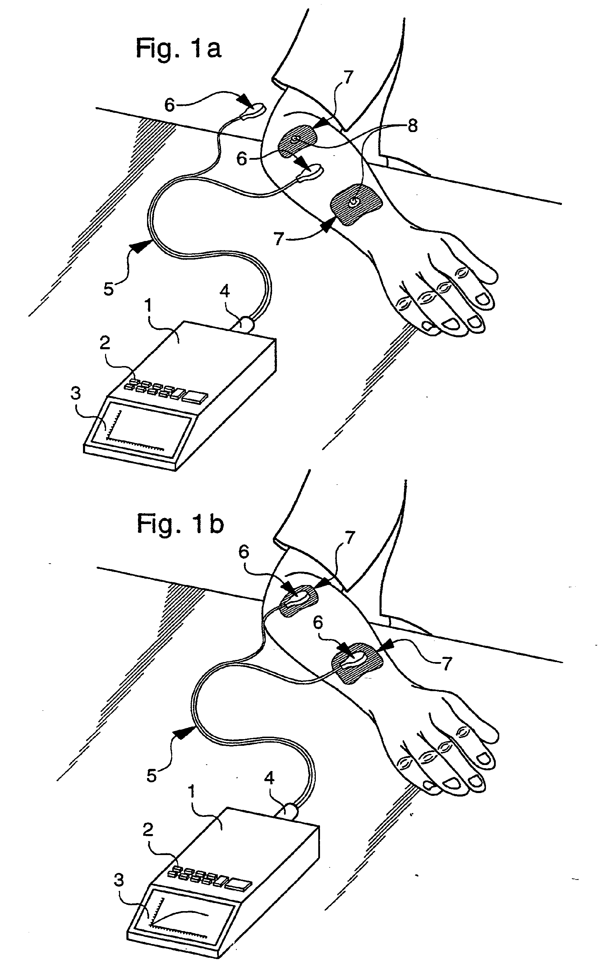

En figures 1a et 1b, le stimulateur est représenté par un boîtier 1 enfermant notamment le générateur d'impulsions de courant et les moyens de réception de signaux provenant du capteur. Sur ledit boîtier 1, des boutons 2 de sélection de programmes servent à choisir le mode d'entraînement souhaité en fonction du sport pratiqué habituellement ou le programme de stimulation en fonction de l'état pathologique (amyotrophie, hypotonie, ...) du muscle à rééduquer. Le stimulateur comprend encore un écran de visualisation 3 pour afficher notamment les programmes choisis, les impulsions de stimulation, la réponse des mesures de réaction musculaire, ou encore des statistiques des séances d'entraînement. L'écran 3 est constitué par exemple d'un affichage à cristaux liquides.

Une extrémité 4 d'une paire de câbles électriques 5 est branchée de manière amovible sur une des prises d'entrée et de sortie de signaux du boîtier 1 du stimulateur. D'autres prises pour le branchement du câble sont accessibles pour brancher plusieurs paires de câbles électriques 5. Depuis le branchement à la prise correspondante, les deux câbles sont réunis de façon à ce qu'ils ne se torsadent notamment lors du rangement. Ils sont par contre séparés sur la seconde moitié de la longueur des câbles pour pouvoir fixer leur connecteur 6 aux électrodes 7 espacées. Les connecteurs ont des moyens complémentaires de fixation de manière amovible à des tétons 8 de la structure d'électrode 7.

Dans la structure de l'électrode 7 ou dans le connecteur 6 sont logés des capteurs de mesure des réactions des muscles, non visibles en figure 1a et 1b. Les capteurs sont logés soit dans une des électrodes ou dans un des connecteurs ou dans les deux. La mesure des réactions musculaires se fait normalement à l'endroit où l'impulsion de courant arrive sur l'électrode, car l'autre électrode ne sert que de retour de courant.

Une pile logée dans le boîtier fournit l'alimentation du stimulateur, mais il est concevable également que le stimulateur reçoive une alimentation en tension externe par un branchement sur le réseau 220V ou 110 V via un transformateur.

En figure 1a, les deux connecteurs 6 sont montrés dans une position éloignée des électrodes 7, car dans un premier temps, l'utilisateur place, en général au niveau des points moteurs, les électrodes souples 7 autocollantes avec leur surface active en contact avec la peau. Dans une forme d'exécution, l'autocollant entoure par exemple la surface active qui occupe plus de la moitié de la surface de la structure d'électrode.

Une fois les électrodes posées sur la peau, les connecteurs 6 sont fixés aux électrodes 7, comme on peut le voir en figure 1b. Dans ce mode de réalisation, les connecteurs 6 sont montés libres en rotation sur les tétons 8.

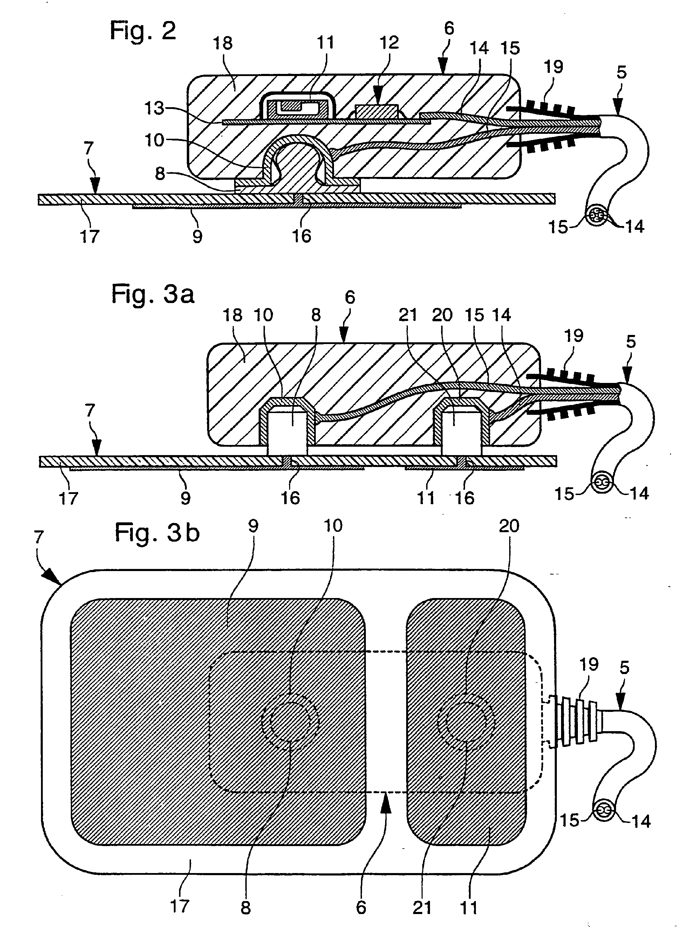

La figure 2 montre un premier mode de réalisation de l'ensemble capteur avec l'électrode de stimulation. Le capteur 11 est inséré dans la masse 18 du connecteur 6 dans le cas où il est obtenu par moulage d'une matière plastique. Dans le connecteur, juste au-dessus des moyens complémentaires de fixation 10 au téton 8 de l'électrode, se trouve un accéléromètre constituant le capteur 11 et disposé sur un circuit imprimé 13 qui comprend tous les composants 12 pour l'amplification et le traitement des signaux de l'accéléromètre. L'accélération obtenue par la vibration des muscles stimulés est de l'ordre de quelques g.

En lieu et place de l'accéléromètre 11, un capteur acoustique, tel qu'un microphone peut être monté sur le circuit imprimé pour pratiquer des mesures de réactions musculaires.

Au moins deux fils conducteurs isolés, de préférence trois fils 14 sont fixés sur des plages métalliques du circuit imprimé pour apporter d'une part l'alimentation électrique provenant du boîtier du stimulateur aux composants du circuit imprimé et d'autre part envoyer à destination du boîtier du stimulateur les signaux de mesure des vibrations musculaires. Un autre fil conducteur 15 isolé est fixé aux moyens métalliques 10 pour amener les impulsions de courant à l'électrode. Tous les fils conducteurs isolés 14 et 15 sont enfermés dans une gaine d'un câble électrique 5.

La structure d'électrode est composée d'un plan de base 17 en matériau souple isolant, tel qu'un tissu ou un élastomère, pouvant épouser, la forme sur laquelle elle est posée, par exemple le bras d'un utilisateur. Au-dessous de la structure 17, un film conducteur est fixée, par exemple par collage ou par dépôt de particules conductrices, sur une grande partie de la surface de la structure 17. Ce film conducteur constitue la surface active 9 de l'électrode par laquelle les impulsions de courant excitent les nerfs-moteurs des muscles à stimuler. Le film métallique 9 est relié au travers d'un trou 16 conducteur, notamment métallisé, pratiqué dans la structure 17 au téton 8 métallique.

Le pourtour de la surface active de l'électrode 9 est enduit d'un matériau autocollant ou recouvert par un film autocollant pour pouvoir maintenir l'électrode 7 sur la peau d'un utilisateur. Ces électrodes sont en principe des électrodes jetables qui peuvent servir pour une séance d'entraînement ou pour plusieurs séances.

Dans une variante de réalisation, la fixation du connecteur à la structure de l'électrode par un bouton à pression peut être inversée en plaçant les moyens complémentaires 10 sur le plan de base 17 et le téton 8 sur le connecteur 6.

Aux figures 3a et 3b, un deuxième mode de réalisation de l'ensemble capteur électrode est présenté. Le capteur utilisé dans ce mode de réalisation est du type électromyographique.

Comme dans la première forme d'exécution discuté ci-devant, le connecteur 6 obtenu par moulage plastique 18 peut comprendre en son intérieur tous les composants électroniques pour le traitement des signaux venant du capteur EMG, mais dans cette variante de la figure 3a, tous les composants électroniques sont intégrés dans le boîtier du stimulateur.

Le connecteur 6 comprend deux pots métalliques 10 et 20 reliés, par exemple par soudure, chacun à l'extrémité d'un fil conducteur isolé respectif 14 et 15, ou inversement. Les pots, ainsi qu'une longueur des fils conducteurs et l'extrémité d'un manchon flexible 19 du câble électrique 5, sont moulés dans la matière plastique 18 du connecteur.

Le câble électrique 5 enferme, dans ce cas, uniquement deux fils conducteurs isolés 14 et 15 dans sa gaine isolante.

La structure de l'électrode comprend, sous le plan de base 17, une première surface conductrice active 9 de l'électrode de stimulation et une deuxième surface conductrice active 11 sans contact avec la première surface active constituant le capteur EMG. La deuxième surface active est placée à côté de la première surface active. Comme représenté à la figure 3b, la première surface active 9 est réalisée par exemple avec une dimension supérieure à la deuxième surface active 11. Autour des surfaces actives, le plan de base est enduit ou recouvert d'un matériau ou d'un film autocollant pour le maintien sur la peau de l'utilisateur sans l'aide d'autres moyens.

En figure 3b, la forme des surfaces actives est approximativement rectangulaire, mais d'autres exécutions sont tout-à-fait concevables, par exemple d'avoir la première surface active 9 sous forme circulaire placée au centre de la structure de l'électrode et la deuxième surface active sous forme d'anneau placée coaxialement à la première surface.

Chaque surface active 9 et 11 est reliée, au travers de trous 16 conducteurs, notamment métallisés, à un téton métallique correspondant 8 et 21 se trouvant de l'autre côté du plan de base. Ces tétons 8 et 21 étant chanfreiné sur leur partie sommitale sont insérés avec une certaine résistance mécanique dans les pots métalliques 10 et 20 du connecteur pour y être maintenus en utilisation. L'insertion à force dans les pots métalliques avec l'aide des chanfreins pour le guidage assure un bon contact électrique pour la transmission des impulsions de courant à l'électrode et la mesure électrique des réactions musculaires. Bien entendu en agencement comme vu en figure 2 peut également s'appliquer dans cette deuxième forme d'exécution.

Plusieurs surfaces actives 9 et 11, que ce soit pour la stimulation électrique ou la mesure, peuvent être placées sous le plan de base 17. Les surfaces actives de stimulation ou de mesure sont soit toutes connectées électriquement en surface du plan de base 17 au travers des trous métallisés 16, soit reliées chacune à un téton correspondant. Dans ce dernier cas, on doit disposer d'un connecteur multipolaire.

Les deux tétons 8 et 21, ainsi que les deux pots métalliques 10 et 20 peuvent être conçus plus rapprochés l'un de l'autre, mais cela implique de réaliser des conducteurs métalliques sur le plan de base 17 du côté du connecteur reliant les trous métallisés 16 avec chacun des tétons 8 et 21.

Il est également concevable de prévoir les tétons 8 et 21 sur le connecteur 6 et les pots métalliques 10 et 20 sur le plan de base 17.

Comme précédemment, les électrodes ont une structure souple pour épouser la surface de la peau où elle est placée, mais rien n'empêche de la concevoir rigide.

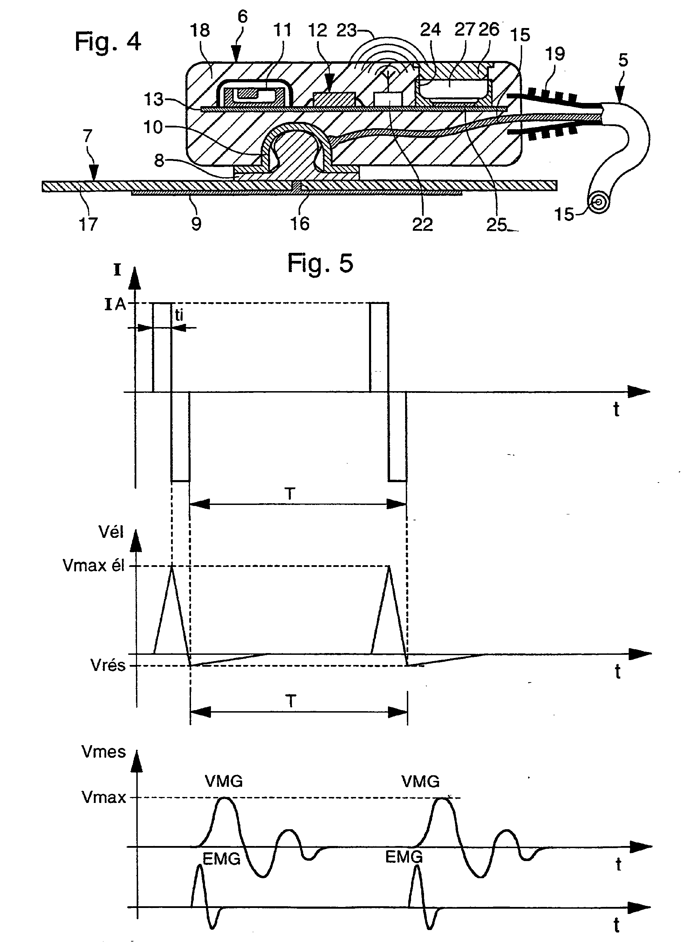

La figure 4 montre une troisième forme d'exécution avec un capteur 11 identique à celui montré en figure 2. Les éléments équivalents à ceux de la figure 2 portent les mêmes signes de référence, et ne seront plus tous expliqués.

Dans cette troisième forme d'exécution, le câble 5 ne comprend dans une gaine isolante qu'un fil conducteur 15 pour amener les impulsions électriques à l'électrode. Les signaux de mesure du capteur 11, traités ou non dans le connecteur 6, sont, par contre, envoyés par des moyens d'émission de signaux 22 de mesure sans fil à l'aide d'ondes élecromagnétiques 23 ou autres à destination de moyens électroniques de réception dans le boîtier du stimulateur. Ces moyens d'émission sont placés sur le circuit imprimé 13 pour recevoir les signaux de mesure du capteur 11. Les ondes 23 captées par les moyens de réception du boîtier sont transformées en signaux électriques représentant les valeurs des mesures du capteur 11 pour être affichées sur un écran et/ou pour adapter les paramères de stimulation.

Une source d'alimentation pour tous les composants électroniques 11, 12 et 22 est prévue dans le connecteur 6 sous forme d'une pile électrique 27. Les pôles positifs et négatifs de la pile 27 sont en contact dans un logement de pile avec une paroi métallique 24 pour un des pôles et avec un fond métallique 25 pour l'autre pôle. Le maintien de la pile dans son logement est assuré par un bouchon 26 poussant la pile 27 contre ses contacts 24 et 25. Ce bouchon 26 est soit vissé, soit inséré à force, soit soudé.

Le bouchon 26 pourrait être oublié, si la pile dans son logement était insérée dans la masse du connecteur 6, dans le cas où il ne serait pas jugé nécessaire de la changer lorsqu'elle serait usagée.

Le connecteur est monté de manière amovible sur les structures d'électrode que ce soit dans la première ou la deuxième ou la troisième formes d'exécution pour permettre à l'utilisateur de d'abord placer les électrodes aux endroits choisis sans être gêné par les câbles électriques. Le connecteur aurait également pu être solidaire de la structure de l'électrode.

Les moyens de fixation du connecteur à l'électrode respective peuvent prendre diverses autres formes que celles mentionnées précédemment. On peut imaginer des moyens de fixation à l'aide d'un aimant logé soit dans le connecteur, soit sur la structure de l'électrode, et d'une partie métallique placée soit sur la structure de l'électrode, soit sur le connecteur. Cet agencement de fixation doit garantir le contact entre des plages métalliques entre les deux éléments pour la fourniture des impulsions électriques ou également pour la mesure du capteur.

La figure 5 montre à titre indicatif trois diagrammes des signaux arrivant aux électrodes de stimulation et ceux tirés de la mesure des réactions ou réponses musculaires que ce soit par un accéléromètre (VMG) ou un capteur électromyographique (EMG).

On impose tout d'abord une impulsion de courant à l'électrode de stimulation. Cette impulsion peut être monophasique, mais de préférence biphasique comme représenté sur la figure 5.

L'amplitude maximum du courant IA s'échelonne de 0 à 120 mA. Plus cette amplitude est élevée et plus le nombre de fibres musculaires recrutées est élevé. Cela correspond donc au recrutement spatial des fibres qui exécutent le travail requis par le programme sélectionné.

Le deuxième diagramme de la figure 5 montre la forme schématique de la tension électrode peau. Cette tension passe par une valeur maximum Vmax aux environs de 100 V et minimum de -10 V. Après que l'impulsion de courant soit revenue à 0, il subsiste une tension résiduelle Vrés de quelques volts sur les électrodes, c'est pourquoi il est difficile d'employer la même surface active pour mesurer à l'aide d'un capteur EMG les variations de tension de réaction des muscles stimulés, car la tension mesurée par le capteur Vmes est de l'ordre de quelques millivolts.

Les variations de tension dues aux vibrations musculaires et mesurées par l'accéléromètre (quelques g) et le capteur EMG pour des impulsions de courant à basse fréquence sont montrées au troisième diagramme.

A plus haute fréquence des impulsions, lorsque le muscle est contracté, l'accéléromètre fournit un signal seulement lors des phases initiales et finales de la contraction. Par contre, le capteur EMG donne un signal même pendant la phase de contraction du muscle.

Ainsi, pour obtenir des mesures au moyen d'un accéléromètre, on peut mesurer le signal d'accélération produit soit par une ou plusieurs secousse musculaires entre les périodes de contractions musculaires, soit par la phase initiale ou finale de la contraction musculaire.

Pour un programme de force, la fréquence des impulsions est élevée, tandis que pour un programme d'endurance cette fréquence est basse. Il faut savoir que pour les fibres musculaires lentes, la fréquence est de 30 Hz, alors que pour les fibres musculaires rapides, elle est de 60 Hz.

Suite à la description qui vient d'être faite, plusieurs autres variantes de réalisation d'un stimulateur avec combinaison d'une électrode avec un capteur de mesure peuvent être imaginées à la portée de l'homme du métier sans sortir du cadre de l'invention.

## Revendications

1. Stimulateur électrique neuromusculaire avec mesure des réactions musculaires générées par des impulsions électriques, comprenant un générateur d'impulsions électriques disposé dans un boîtier du stimulateur (1), au moins une paire d'électrodes de stimulation (7) destinées à être posées sur la peau d'un utilisateur au niveau des points moteurs des muscles à stimuler, chaque électrode (7) étant reliée à une extrémité d'un câble électrique (5) dont l'autre extrémité (4) est raccordée au boîtier pour recevoir des impulsions électriques de générateur, au moins un capteur (11) sensible à des réactions musculaires provoquées par les impulsions électriques de stimulation et agencé pour transmettre des signaux électriques de mesure représentatifs desdites réactions musculaire à des moyens électroniques dans le boîtier du stimulateur et au moins un fil conducteur (15) par câble électrique (5) connectant l'électrode (7) respective indépendamment du capteur (11) **caractérisé en ce que** le capteur (11) est lié mécaniquement à une des électrodes (7) en étant placé dans la structure de celle-ci ou à l'extrémité d'un des câbles (5) côté électrode en étant placé dans un connecteur (6) reliant le câble à l'électrode.

2. Stimulateur selon la revendication 1, **caractérisé en ce que** chaque extrémité des câbles électriques côté électrode est fixée solidairement à la structure de l'électrode (17) respective par le connecteur.

3. Stimulateur selon la revendication 1, **caractérisé en ce que** chaque extrémité de câble côté électrode a un connecteur (6) relié à la structure de l'électrode (17) respective par des moyens de fixation amovible.

4. Stimulateur selon la revendication 3, **caractérisé en ce que** les moyens de fixation amovible sont du type à bouton-pression (8, 10) servant également de contact électrique entre le connecteur et au moins une surface active conductrice (9) de l'électrode respective.

5. Stimulateur selon la revendication 3, **caractérisé en ce que** les moyens de fixation amovible, servant également de contact électrique entre le connecteur (6) et au moins deux surfaces actives de l'électrode (7), comportent au moins deux tétons conducteurs (8, 21) insérés avec une certaine résistance mécanique dans deux pots conducteurs (10, 20), les tétons (8, 21) faisant partie de la structure de l'électrode (17) et les pots (10, 20) faisant partie du connecteur (6), ou inversement.

6. Stimulateur selon l'une des revendications 1, 2, 3 et 5, **caractérisé en ce que** le capteur de mesure est un capteur électromyographique (11) ayant au moins une surface active conductrice placée sans contact électrique à côté d'au moins une autre surface active conductrice (9) de l'électrode recevant les impulsions électriques, lesdites surfaces actives étant posées sur les points moteurs des muscles à stimuler.

7. Stimulateur selon l'une des revendications 1 à 5, **caractérisé en ce que** le capteur est un accéléromètre (11) ou un microphone intégré dans le connecteur (6) de l'extrémité du câble côté électrode ou dans la structure d'une des électrodes (17) respectives.

8. Stimulateur selon l'une des revendications 6 et 7, **caractérisé en ce que** des moyens de traitement des signaux (12) reçus du capteur sont intégrés dans le connecteur (6) ou dans la structure de l'électrode (7).

9. Stimulateur selon l'une des revendications 1, 6, 7 et 8, **caractérisé en ce que** le capteur (11) est en communication avec les moyens électroniques du stimulateur par des moyens d'émission et/ou de réception de signaux (22, 23) sans fil logés dans le connecteur (6) de câble ou dans la structure de l'électrode (7), ou par au moins un fil conducteur (14) du câble autre que celui de l'électrode (7).

10. Stimulateur selon la revendication 9, **caractérisé en ce qu'**une source d'alimentation électrique est logée dans le connecteur (6) ou dans la structure de l'électrode (7) pour alimenter les composants électroniques de mesure des réactions musculaires.

11. Stimulateur selon l'une des revendications précédentes, **caractérisé en ce qu'**il comprend, sur le boîtier (1), un écran de visualisation (3) capable d'afficher notamment des programmes de stimulation électrique et des informations relatives aux signaux électriques de mesure des réactions musculaires.

12. Câble électrique pour un stimulateur selon la revendication 1, comprenant dans une gaine isolante au moins un fil conducteur (15) pour connecter la ou les surfaces actives de l'électrode (7) de stimulation indépendamment d'un capteur (11), **caractérisé en ce qu'**une extrémité du câble (5) côté électrode a un connecteur (6) pour être relié à la structure d'une électrode de stimulation (17) par des moyens de fixation amovible servant également de contact électrique pour la ou les surfaces actives de l'électrode, **en ce que** le connecteur (6) comprend au moins une partie du capteur (11) sensible à des réactions musculaires.

13. Câble électrique selon la revendication 12, **caractérisé en ce que** le connecteur enferme des moyens de traitement de signaux (12) délivrés par le capteur.

14. Câble électrique selon la revendication 12, **caractérisé en ce que** les moyens de fixation avec l'électrode sont du type à bouton-pression (8, 10) ou du type multibroches (8, 10; 20, 21).

15. Câble électrique selon l'une des revendications 12 et 13, **caractérisé en ce que** le connecteur (6) comprend des moyens d'émission et/ou de réception de signaux (22, 23) sans fil, et une source d'alimentation électrique (27) des composants électroniques (11, 12, 22) de mesure des réactions musculaires.

## Claims

1. Neuromuscular electrical stimulator with measurement of the muscle reactions generated by electrical pulses, comprising an electrical pulse generator positioned in a stimulator unit (1), at least one pair of stimulation electrodes (7) which are intended to be positioned on the skin of a user at the motor points of the muscles that are to be stimulated, each electrode (7) being connected to one end of an electrical lead (5) the other end (4) of which is connected to the unit in order to receive electrical pulses from the generator, at least one sensor (11) sensitive to muscle reactions caused by the electrical stimulation pulses and designed to transmit electrical measurement signals representative of the said muscle reactions to electronic means in the stimulator unit and at least one conducting wire (15) per electrical lead (5) connecting the respective electrode (7) independently of the sensor (11), **characterized in that** the sensor (11) is mechanically connected to one of the electrodes (7) by being placed within the structure thereof or at the electrode end of one of the leads (5) by being placed in a connector (6) that connects the lead to the electrode.

2. Stimulator according to Claim 1, **characterized in that** each electrode end of the electrical leads is fixed securely to the structure of the respective electrode (17) by the connector.

3. Stimulator according to Claim 1, **characterized in that** each electrode end of a lead has a connector (6) connected to the structure of the respective electrode (17) by removable attachment means.

4. Stimulator according to Claim 3, **characterized in that** the removable attachment means are of the press stud type (8, 10) also used for electrical contact between the connector and at least one conducting active surface (9) of the respective electrode.

5. Stimulator according to Claim 3, **characterized in that** the removable attachment means, which are also used for electrical contact between the contactor (6) and at least two active surfaces of the electrode (7), comprise at least two conducting pegs (8, 21) inserted against a certain degree of mechanical resistance into two conducting receptacles (10, 20), the pegs (8, 21) being part of the structure of the electrode (17) and the receptacles (10, 20) forming part of the connector (6), or vice versa.

6. Stimulator according to one of Claims 1, 2, 3 and 5, **characterized in that** the measurement sensor is an electromyographic sensor (11) having at least one conducting active surface positioned without electrical contact beside at least one other conducting active surface (9) of the electrode that receives the electrical pulses, the said active surfaces being positioned on the motor points of the muscles that are to be stimulated.

7. Stimulator according to one of Claims 1 to 5, **characterized in that** the sensor is an accelerometer (11) or a microphone built into the connector (6) at the electrode end of the lead or into the structure of one of the respective electrodes (17).

8. Stimulator according to one of Claims 6 and 7, **characterized in that** signal processing means (12) for processing the signals received from the sensor are built into the connector (6) or into the structure of the electrode (7).

9. Stimulator according to one of Claims 1, 6, 7 and 8, **characterized in that** the sensor (11) is in communication with the electronic means of the stimulator via wireless signal transmitting and/or receiving means (22, 23) housed in the lead connector (6) or in the structure of the electrode (7), or via at least one conducting wire (14) of the lead other than the wire for the electrode (7).

10. Stimulator according to Claim 9, **characterized in that** an electrical power supply is housed in the connector (6) or in the structure of the electrode (7) to power the electronic components involved in measuring the muscle reactions.

11. Stimulator according to one of the preceding claims, **characterized in that** it comprises, on the unit (1), a display screen (3) capable in particular of displaying the electrical stimulation programs and information relating to the electrical signals used to measure the muscle reactions.

12. Electrical lead for a stimulator according to Claim 1, comprising an insulating sheath and at least one conducting wire (15) for connecting the active surface or surfaces of the stimulation electrode (7) independently of a sensor (11), **characterized in that** an electrode end of the lead (5) has a connector (6) to be connected to the structure of a stimulation electrode (17) by removable attachment means that also provide electrical contact for the active surface or surfaces of the electrode, **in that** the connector (6) comprises at least part of the sensor (11) sensitive to muscle reactions.

13. Electrical lead according to Claim 12, **characterized in that** the connector contains signal processing means (12) for processing signals delivered by the sensor.

14. Electrical lead according to Claim 12, **characterized in that** the means of attachment to the electrode are of the press stud type (8, 10) or of the multi-pin type (8, 10; 20, 21).

15. Electrical lead according to one of Claims 12 and 13, **characterized in that** the connector (6) comprises wireless signal transmitting and/or receiving means (22, 23) and an electrical power supply (27) for powering the electronic components (11, 12, 22) involved in measuring the muscle reactions.

## Patentansprüche

1. Neuromuskulärer elektrischer Stimulator mit Messung der Muskelreaktionen, die durch elektrische Impulse hervorgerufen werden, mit einem Generator für elektrische Impulse, der in einem Stimulatorgehäuse (1) angeordnet ist, wenigstens einem Paar Stimulationselektroden (7), die dazu bestimmt sind, auf die Haut eines Anwenders auf Höhe der Reizpunkte der zu stimulierenden Muskeln gelegt zu werden, wobei jede Elektrode (7) mit einem Ende eines elektrischen Kabels (5) verbunden ist, dessen anderes Ende (4) mit dem Gehäuse verbunden ist, um elektrische Impulse des Generators zu empfangen, wenigstens einem Sensor (11), der für Muskelreaktionen empfindlich ist, die durch die elektrischen Stimulationsimpulse hervorgerufen werden, und so ausgelegt ist, dass er elektrische Messsignale, die die Muskelreaktionen repräsentieren, an elektronische Mittel in dem Stimulatorgehäuse überträgt, und wenigstens einem Leiterdraht (15) pro elektrischem Kabel (5), der die jeweilige Elektrode (7) unabhängig vom Sensor (11) verbindet, **dadurch gekennzeichnet, dass** der Sensor (11) mit einer der Elektroden (7) mechanisch verbunden ist, indem er in deren Struktur angeordnet ist, oder mit dem Ende eines der Kabel (5) auf Seiten der Elektrode mechanisch verbunden ist, indem er in einem das Kabel mit der Elektrode verbindenden Verbinder (6) angeordnet ist.

2. Stimulator nach Anspruch 1, **dadurch gekennzeichnet, dass** jedes Ende der elektrischen Kabel auf Seiten der Elektrode mit der jeweiligen Elektrodenstruktur (17) durch den Verbinder fest verbunden ist.

3. Stimulator nach Anspruch 1, **dadurch gekennzeichnet, dass** jedes Kabelende auf Seiten der Elektrode einen Verbinder (6) besitzt, der mit der jeweiligen Elektrodenstruktur (17) durch lösbare Befestigungsmittel verbunden ist.

4. Stimulator nach Anspruch 3, **dadurch gekennzeichnet, dass** die Mittel zum lösbaren Befestigen vom Typ Druckknopf (8, 10) sind, die außerdem als elektrischer Kontakt zwischen dem Verbinder und wenigstens einer aktiven leitenden Oberfläche (9) der jeweiligen Elektrode dienen.

5. Stimulator nach Anspruch 3, **dadurch gekennzeichnet, dass** die Mittel zum lösbaren Befestigen, die außerdem als elektrischer Kontakt zwischen dem Verbinder (6) und wenigstens zwei aktiven Oberflächen der Elektrode (7) dienen, wenigstens zwei Leiterstifte (8, 21) aufweisen, die entgegen einem bestimmten mechanischen Widerstand in zwei Leiterbuchsen (10, 20) eingesteckt sind, wobei die Stifte (8, 21) einen Teil der Struktur der Elektrode (17) bilden und die Buchsen (10, 20) einen Teil des Verbinders (6) bilden oder umgekehrt.

6. Stimulator nach einem der Ansprüche 1, 2, 3 und 5, **dadurch gekennzeichnet, dass** der Messsensor ein elektromyographischer Sensor (11) ist und wenigstens eine aktive leitende Oberfläche besitzt, die ohne elektrischen Kontakt auf der Seite wenigstens einer anderen aktiven leitenden Oberfläche (9) der die elektrischen Impulse empfangenden Elektrode angeordnet ist, wobei die aktiven Oberflächen an den Reizpunkten der zu stimulierenden Muskeln aufgelegt sind.

7. Stimulator nach einem der Ansprüche 1 bis 5, **dadurch gekennzeichnet, dass** der Sensor ein Beschleunigungsmesser (11) oder ein Mikrophon ist, der bzw. das in den Verbinder (6) des Kabelendes auf Seiten der Elektrode oder in die Struktur einer der jeweiligen Elektroden (17) integriert ist.

8. Stimulator nach einem der Ansprüche 6 und 7, **dadurch gekennzeichnet, dass** die Verarbeitungsmittel (12) für die vom Sensor empfangenen Signale in den Verbinder (6) oder in die Struktur der Elektrode (7) integriert sind.

9. Stimulator nach einem der Ansprüche 1, 6, 7 und 8, **dadurch gekennzeichnet, dass** der Sensor (11) mit den elektronischen Mitteln des Stimulators durch Mittel (22, 23) zum drahtlosen Senden und/oder Empfangen von Signalen, die sich in dem Verbinder (6) des Kabels oder in der Struktur der Elektrode (7) befinden, oder durch wenigstens einen Leiterdraht (14) des Kabels, der von jenem der Elektrode (7) verschieden ist, kommuniziert.

10. Stimulator nach Anspruch 9, **dadurch gekennzeichnet, dass** sich eine Stromversorgungsquelle in dem Verbinder (6) oder in der Elektrodenstruktur (7) befindet, um die elektronischen Komponenten zum Messen der Muskelreaktionen zu versorgen.

11. Stimulator nach einem der vorhergehenden Ansprüche, **dadurch gekennzeichnet, dass** er an dem Gehäuse (1) einen Anzeigeschirm (3) aufweist, der insbesondere Programme zur elektrischen Stimulation und Informationen bezüglich der elektrischen Messsignale der Muskelreaktionen anzeigen kann.

12. Elektrisches Kabel für einen Stimulator nach Anspruch 1, das in einer Isolierhülle wenigstens einen Leiterdraht (15) für den Anschluss der aktiven Oberfläche(n) der Stimulationselektrode (7) unabhängig von einem Sensor (11) enthält, **dadurch gekennzeichnet, dass** ein Ende des Kabels (5) auf Seiten der Elektrode einen Verbinder (6) besitzt, um mit der Struktur einer Stimulationselektrode (17) durch Mittel zum lösbaren Befestigen verbunden zu werden, die außerdem als elektrischer Kontakt für die aktive(n) Oberfläche(n) der Elektrode dienen, und dass der Verbinder (6) wenigstens einen Sensorabschnitt (11) enthält, der für die Muskelreaktionen empfindlich ist.

13. Elektrisches Kabel nach Anspruch 12, **dadurch gekennzeichnet, dass** der Verbinder Mittel (12) zum Verarbeiten von Signalen, die von dem Sensor geliefert werden, umschließt.

14. Elektrisches Kabel nach Anspruch 12, **dadurch gekennzeichnet, dass** die Mittel für die Befestigung mit der Elektrode vom Typ Druckknopf (8, 10) oder vom Typ Mehrfachstift (8, 10; 20, 21) sind.

15. Elektrisches Kabel nach einem der Ansprüche 12 und 13, **dadurch gekennzeichnet, dass** der Verbinder (6) Mittel (22, 23) zum drahtlosen Senden und/oder Empfangen von Signalen und eine Stromversorgungsquelle (27) für die elektronischen Komponenten (11, 12, 22) zum Messen von Muskelreaktionen enthält.
